# EUROPÄISCHE PATENTSCHRIFT

(11) **EP 2 566 414 B1**
(45) Veröffentlichungstag und Bekanntmachung des Hinweises auf die Patenterteilung: **02.08.2017**
(21) Anmeldenummer: 11718978.7
(22) Anmeldetag: 04.05.2011
(51) Int. Cl.: A61F 2/04

(54) **HARNBLASENPROTHESE FÜR DIE SUBKUTANE IMPLANTATION**
BLADDER PROSTHESIS FOR SUBCUTANEOUS IMPLANTATION
PROTHÈSE DE VESSIE DESTINÉE À UNE IMPLANTATION SOUS-CUTANÉE

(30) Priorität: 06.05.2010 DE 102010028675
(43) Veröffentlichungstag der Anmeldung: 13.03.2013
(73) Patentinhaber: Primed Halberstadt Medizintechnik GmbH, 38820 Halberstadt (DE)
(72) Erfinder: LIEHR, Uwe-Bernd, 39104 Magdeburg (DE)
(74) Vertreter: Kailuweit & Uhlemann Patentanwälte Partnerschaft mbB
(86) Internationale Anmeldenummer: PCT/EP2011/057146
(87) Internationale Veröffentlichungsnummer: WO 2011/138371

(56) Entgegenhaltungen:
- EP-A2- 0 972 496
- EP-A2- 1 020 165
- WO-A1-2009/077047
- WO-A2-2007/095193
- FR-A5- 2 116 838
- US-A- 4 969 902
- US-A- 5 041 136

## Beschreibung

Die Erfindung betrifft eine Harnblasenprothese für die subkutane Implantation, welche eine kontinente Harnableitung ermöglicht. Die Erfindung findet ihre Anwendung in der Medizin als Ersatz der Harnblase, welche krankheitsbedingt oder aufgrund von Zerstörungen entfernt wurde oder funktionsunfähig ist.

Krankheitsbedingt kann es erforderlich sein, eine totale Entfernung der Harnblase vorzunehmen. Dies ist insbesondere bei Krebserkrankungen im kleinen Becken oder im Bauchraum der Fall. Oft werden Tumore dort erst in einem Zustand entdeckt, in dem sie fortgeschritten und daher nicht mehr problemlos zu entfernen sind. In solchen Fällen sind größere Operationen und Nachbestrahlungen oder Mehrfachoperationen unausweichlich, um ein erneutes Tumorwachstum zu stoppen oder zumindest zu verzögern. Liegen diese Krebsgeschwüre im Bereich des kleinen Beckens vor, z. B. bei Tumoren der Gebärmutter, der Prostata oder des unteren Darmes, sind oft auch die Harnleiter und die Harnblase betroffen.

Harnleiter sind empfindliche gefäßähnliche Strukturen, welche im hinteren Bauchraum von den Nieren zur Harnblase verlaufen. Die Harnleiter können durch einen wachsenden Tumor gedrückt oder auch zerstört werden. Nach größeren Krebsoperationen oder Strahlenbehandlungen besteht außerdem die Gefahr, dass die Harnleiter durch Narbengewebe verengt werden, so dass sie den Urin nicht mehr adäquat in die Harnblase ableiten können.

Eine totale Entfernung der Harnblase ist beispielsweise bei Tumoren erforderlich, welche von der Harnblase selbst ausgehen oder bei Tumoren der umgebenden Organe wie insbesondere Darm, Gebärmutter oder Scheide, bei denen bereits eine Invasion in die Harnblase erfolgte.

In Fällen, bei denen ausschließlich die Harnblase von der Erkrankung betroffen ist, wird die originäre Harnblase entweder durch autologe, aus der Darmkontinuität ausgeschaltete Darmbestandteile ersetzt, oder ein ausgeschaltetes Darmsegment in die Haut als inkontinentes Urostoma implantiert. Ersteres Verfahren stellt als kontinente Versorgungsvariante an den betroffenen Patienten relativ klar formulierte Anforderungen. Es müssen tumorchirurgische sowie anatomische Voraussetzungen erfüllt sein. Vor-oder Nachbehandlungen, wie Strahlentherapien oder erneute chirurgische Eingriffe im Neoblasen-Operationsgebiet limitieren dieses OP-Verfahren. In den Fällen mit fortgeschrittenen Tumorbefunden oder auch der Befall von Nachbarorganen oder -strukturen sind orthotope Harnblasenersatzoperationen wie oben beschrieben ebenfalls kein Standard. Dies trifft auch für Tumorentitäten anderer Fachrichtungen, wie der Chirurgie oder Gynäkologie mit Harnblaseninfiltration zu. Auch hier sind nur inkontinente Harnableitungsverfahren möglich.

In diesen Fällen werden nach einer Totalentfernung der Harnblase bisher Harnableitungen implantiert, die mit extrakorporalen Auffangsystemen verbunden sind und den Harn inkontinent ableiten (z. B. perkutane Nephrostomien oder Urinstoma mit Auffangbeuteln). Dies bedeutet eine schwerwiegende Einschränkung der Lebensqualität der Patienten. Die regelmäßig und kurzfristig zu erneuernden Ableitungs- und Auffangsysteme bedeuten hohen Aufwand und Kosten.

Eine Harnblasenprothese, welche bei einer einfachen Anwendung einen kontinenten Ablauf ermöglicht, kann diese Nachteile überwinden.

Es sind Lösungen bekannt, eine künstliche Harnblase bereitzustellen, welche an die natürlichen Harnleiter und Harnröhre angeschlossen wird.

FR 2 116 838 offenbart eine künstliche Harnblase, die an zwei Harnleiter und an die Harnröhre eines Patienten angeschlossen werden soll. Diese Harnblase ist mit einem System von drei inneren Klappenventilen ausgestattet, von denen zwei an den Harnleitern den Zustrom gewährleisten, während ein drittes gegenüber den zwei Klappenventilen gegensinnig arbeitet (Ablass). Ferner ist die künstliche Harnblase mit einer Steuerungseinrichtung für die Klappe ausgestattet, welche die Steuerung über ein Gas, welches sich in einem gesonderten System befindet, vermittelt. Weiter enthält das System eine Kontrollvorrichtung zur Kontrolle des Drucks in der Harnblase.

FR 2 255 877 offenbart eine implantierbare Harnblasenprothese, die für den Anschluss an die Harnleiter und die Harnröhre eines Patienten geeignet ist. Alternativ kann der Anschluss an die Nieren auch über Harnleiterprothesen erfolgen. Die Harnblasenprothese kann alternativ auch ohne Harnröhre direkt an die abdominale oder perineale Scheidewand des Patienten angeschlossen werden. Die offenbarte Harnblasenprothese soll die Hauptfunktionen einer natürlichen Harnblase gewährleisten und auf natürliche Weise gesteuert werden. Die beschriebene Prothese weist keine inneren, d. h. beim Patienten eingesetzten, Ventilklappen auf. Die offenbarte Harnblasenprothese ist verformbar und wird dadurch beim Eintritt des Harns gespannt und entspannt sich während des Ablassens.

Problematisch bei den in FR 2 116 838 als auch in FR 2 255 877 vorgeschlagenen Prothesen ist die Verbindung zwischen natürlichen Harnleitern und Harnröhre mit dem künstlichen System. Bei der Verbindung von Humangewebe mit eigener Durchblutung mit artifiziellen Systemen tritt häufig eine ungenügende Verheilung auf, so dass undichte Stellen die Folge sind. Dies stellt ebenso wie Abwehrreaktionen des menschlichen Gewebes gegenüber den künstlichen Systemen ein lebensbedrohliches Gefährdungspotential für den Patienten dar. Mögliche Resultate sind peritonitische Zustände mit Mehrfachorganstörungen im Bauchraum. Operative Eingriffe zur Behebung dieser Störungen stellen per se schwerwiegende und risikobehaftete Eingriffe dar.

Weiter sind Lösungen bekannt, eine künstliche Harnblase bereitzustellen, welche an künstliche Harnleiter angeschlossen werden soll.

DE 199 12 472 A1 und DE 100 46 027 A1 offenbaren künstliche Harnableitungssysteme zum Anschluss an künstliche Harnleiter, welche zur Implantation in den Bauchraum vorgesehen sind. Es werden Systeme bereitgestellt, welche während der Operation je nach individuellem Bedarf an den Patienten angepasst werden können. Künstliche Harnableitungssysteme sollen dadurch ohne vorherige direkte oder indirekte Bestimmung des zur Verfügung stehenden Volumens für die künstliche Harnableitungseinrichtung anpassbar sein, so dass während der Operationsphase erst das in der betreffenden Person zur Verfügung stehende Volumen ermittelt und bestmöglich ausgenutzt wird.

DE 101 56 558 A1 offenbart ein Verschlusssystem und ein hierzu geeignetes Verfahren zum wahlweisen Öffnen und Schließen eines rohrförmigen Körperorgans, z. B. einer künstlichen Harnblase. Das Verschlusssystem umfasst ein Schließelement sowie ein das Schließelement steuerndes Regelungssystem. Das Regelungssystem stellt einen ersten Zustand des Verschlusssystems ein und führt eine Abweichung von dem ersten Zustand selbstregulierend darauf zurück, um in der zu verschließenden Kunstharnblase auftretenden Druckerhöhungen kurzfristig entgegenzuwirken. Dadurch sollen potentielle Beschädigungen der künstlichen Harnblase vermieden werden, die aus einem zu hohen Behälterinnendruck resultieren. Zum implantierbaren Verschlusssystem gehören Sensorelemente, Zuführungsleitungen, eine Pumpeinrichtung, Absperrventile und eine ebenfalls implantierte Energiequelle für den sicheren Betrieb des Systems.

Ein ähnlicher Lösungsansatz wird durch DE 102 39 309 A1 offenbart. Darin wird ein Verfahren zur elektronischen Steuerung eines artifiziellen feinsensorischen Sphinkterimplantats beschrieben. Durch das für dieses Verfahren bereitgestellte System wird mindestens ein Sensorsignal in einer Schaltung umgewandelt und mit Referenzwerten verglichen, wobei sich das Sensorsignal analog zur Differenz zwischen Blaseninnendruck und Cuffdruck verändert. Dadurch wird eine Aktorik gesteuert, die den Innendruck des Behälters reguliert. Dies wird so bewerkstelligt, dass sich der Cuffdruck bzw. der Differenzdruck zwischen Cuff und Harnblase entweder in einem engen Bereich bewegt, welche durch definierte Schwellenwerte begrenzt ist oder dass der Cuffdruck oberhalb bzw. bei Entleerung des Implantats unterhalb eines bestimmten Sicherheitsdrucks liegt. Die beschriebene Lösung stellt ein modular aufgebautes System dar, welches aus Reservoir- und Steuerungsmodulen sowie aus beweglichen Bauteilen in verschiedenen Größen besteht. Sobald das Harnblasenimplantat gefüllt ist, wird eine Vibration ausgelöst. Durch dieses System wird eine manuelle Entleerung der künstlichen Harnblase ermöglicht. Das System enthält Energiespeicher, welche nach der Implantation durch ein externes Gerät aufgeladen werden können, welches auf den Unterbauch aufgelegt wird.

Die vorstehend beschriebenen Lösungen erfordern die Implantation einer künstlichen Harnblase in den unteren Bauchraum, genau an der Stelle, an der sich die originäre Harnblase befand. Beim Entfernen der Harnblase wird die Mitentfernung des unteren Bauchfells notwendig, so dass sich keine getrennten Kompartimente (unterer Bauchraum - Bauchraum) erhalten lassen. Dies führt dazu, dass die in den Bauchraum eingesetzten artifiziellen Systeme prinzipiell Kontakt mit den sensiblen Organen des Bauchraumes haben. Revisionseingriffe bei technischen Störungen von Harnblasenimplantaten, welche in den Bauchraum implantiert wurden, sind mit hohen Risiken für den Patienten behaftet.

Ein weiterer Nachteil an den vorbekannten Lösungen ist, dass das Ablassen des Harns hohe technische Anforderungen an die betroffenen Patienten stellt und daher für einen Großteil der Patientengruppe nicht bedienbar ist.

EP 0 818 978 B1 offenbart ein Harnblasenprothesen-System für die subkutane Implantation, welche aus mehreren Komponenten zusammengesetzt ist. Die umfasst ein Beutelelement aus biokompatiblem Material und ein Ventilelement für die Steuerung des Zulaufs und Ablaufs des Harns zu dem Beutelelement. Der Zu- und Ablauf des Harns wird in dieser Apparatur über ein einziges, komplex gestaltetes, verschließbares- und den Ablauf sicherstellendes Ventilelement gewährleistet. Für Aufbau und Bedienung des in EP 0 818 978 B1 offenbarten Systems ist sowohl seitens des Operateurs als auch seitens des Patienten ein hohes technisches Verständnis erforderlich.

DE 199 00 940 A1 offenbart eine subkutan implantierbare künstliche Blase. Die künstliche Blase besteht aus einem Material, welches ein körperverträglicher elastischer Kunststoff ist, und ist mit zwei Kammern ausgestaltet, welche miteinander über ein Rückschlagventil verbunden sind. Die Kammern haben jeweils einen Boden, der durch eine Folienwand gebildet wird. Die erste Kammer ist mit einer Niere verbunden, die andere Kammer mit dem Ablauf. Die Harnflüssigkeit wird durch die künstliche Blase angesaugt (selbstansaugendes System). Wenn sich die erste Kammer mit Flüssigkeit gefüllt hat, öffnet sich das zwischen den beiden Kammern befindliche Rückschlagventil und erlaubt den Flüssigkeitsfluss in die zweite Kammer. Die Entleerung erfolgt durch manuellen Druck auf die Kammer bzw. durch Pumpwirkung.

DE 198 31 698 C2 offenbart ein Rückschlagventil für eine Harnblasenprothese und eine Anordnung für eine künstliche Harnableitung. Dabei ist jede Niere mit einem eigenen Reservoir verknüpft. Die Reservoirs sind ausgangsseitig mit einem Y-Stück verbunden, welches in eine künstliche oder natürliche Harnröhre mündet und das Rückschlagventil enthält. Die Entleerung der Reservoirs erfolgt durch Druck auf dieselben.

DE 698 21 117 T2 offenbart eine subkutan implantierbare Harnblasenprothese aus einem zweischichtigen Material, welche ein zentrales Reservoir aufweist, an welchem rohrförmige Elemente als künstliche Harnleiter und künstliche Harnröhre angebracht sind. Innerhalb der künstlichen Harnröhre ist ein künstlicher Harnröhrenschließmuskel angebracht, welcher sich nach Implantation vor dem Schambein befindet. Dieser ist ein unterschiedlich gestaltbares Ventilelement, welches durch Druck auf das Reservoir geöffnet wird.

Das Reservoir ist in DE 698 21 117 T2 so gestaltet, dass die zwei Materialien für Innen- und Außenwand des Reservoirs nicht verbunden sind, um eine Austauschbarkeit des inneren Flüssigkeitsbeutels zu ermöglichen. Eine Austauschbarkeit des inneren Flüssigkeitsbeutel erfordert dabei jedoch ein aufwändiges Lösen von mindestens drei Fixationsstellen am Außenmaterial (Zulauf- und Ablauföffnungen). Beim Entleeren des Reservoirs kann sich der innere Flüssigkeitsbeutel in diesem Aufbau nur Zusammenziehen, wenn Luft zwischen die beiden Materialschichten eindringt. Da dies unmöglich ist, verbleibt der Flüssigkeitsbeutel durch Adhäsion an der Außenwand haftend, so dass daraus eine unvollständige Entleerung resultieren kann.

Der durch das Entleeren der Harnblasenprothese erzeugte negative Druck innerhalb des Reservoirs verhindert einen Rückfluss des Harns zu den Nieren, so dass keine komplexen Rückschlagventile an der Verbindung von künstlichen Harnleitern und Reservoir angebracht sind. Durch die in DE 698 21 117 T2 vorgeschlagene Gestaltung wird nicht sichergestellt, dass bei Ausüben von Druck auf das Reservoir dieser zu den druckempfindlichen Nieren transmittiert wird, was eine Hydronephrose oder gar eine Niereninsuffizienz zur Folge haben kann. Bei der Entleerung der Harnblasenprothese, welche durch Druck auf das Reservoir erfolgt, ist nicht sichergestellt, dass kein Druck zu den Nieren übertragen wird bzw. Harn aus dem Reservoir zu den Nieren zurückgedrückt wird.

Die Gestaltung des künstlichen Harnröhrenschließmuskels inmitten des rohrförmigen Elements für die künstliche Harnröhre und der Implantation vor dem Schambein soll ggf. eine Kompression der künstlichen Harnröhre erlauben. Die Erreichbarkeit des Harnröhrenschließmuskels ist hier insbesondere bei adipösen Patienten erschwert, so dass Bedarf an weiteren Entleerungsvarianten besteht, um subkutan implantierbare Harnblasenprothesen universell einzusetzen. Weiter muss unbedingt verhindert werden, dass ein Rückfluss von Harn zu den Nieren möglich ist.

US 4,969,902 A offenbart einen aus mehreren Materialschichten bestehenden künstlichen Darmausgang mit entsprechenden Öffnungen. Die Lösung soll auch auf eine Harnblasenprothese anwendbar sein, würde aber aufgrund des Materialschichtaufbaus zu Problemen beim Entleeren der Harnblase führen.

EP 0 972 496 A2 offenbart einen Implantathalter für Körperimplantate, z.B. eine künstliche Harnblase. Der Implantathalter weist eine Abschirmwand (21) auf, die an einer Seite aus einem gewebefreundlichen Fasermaterial (25) zum Anwachsen körpereigener Zellen besteht und an der anderen Seite eine glatte Anlagefläche (23) für das Implantat (10) aufweist. Der Implantathalter verhindert, daß das Körpergewebe unmittelbar an dem Implantat anwächst, was ein Auswechseln des Implantats (10) erschweren würde. Zum Auswechseln wird die Haut (16) des Patienten aufgeschnitten und die Halterung (26) geöffnet. Der Implantathalter verbleibt im Körper, während das Implantat (10) gewechselt wird.

Die in DE 199 00 940 A1, DE 198 31 698 C und DE 698 21 117 T2 vorgeschlagenen Lösungen für subkutan implantierbare Harnblasenprothesen basieren auf einer Entleerung der Harnblasenprothese durch Druck auf das Reservoir. Dadurch kann nicht immer eine kontinente Harnableitung sichergestellt werden, da in diesen Lösungen die Entleerung auch bei unvorhergesehenem Druck auf die Harnblasenprothese, z. B. durch Husten, Niesen oder Lachen, ausgelöst würde und somit wieder eine Inkontinenz aufträte. Um eine kontinente Ableitung zu ermöglichen, muss jedoch vermieden werden, dass die Harnblasenprothese unkontrolliert geleert wird. Entsprechend besteht Bedarf an Harnblasenprothesen, die so gestaltet sind, dass die Entleerung nicht unkontrolliert ausgelöst wird.

Aufgabe der Erfindung ist es eine Harnblasenprothese bereitzustellen, die für die subkutane Implantation geeignet ist und die in einer einfachen Bedienung eine kontinente Harnableitung ermöglicht. Weiterhin soll die Harnblasenprothese so gestaltet sein, dass sie im Rahmen einer minimalinvasiven Operation implantiert werden kann und einfach operativ ausgetauscht werden kann.

Die Aufgabe wird erfindungsgemäß gelöst durch eine Harnblasenprothese mit den Merkmalen nach Anspruch 1 sowie einen Bausatz zur Herstellung einer Harnblasenprothese nach Anspruch 9. Weitere Ausgestaltungen enthalten die Unteransprüche 2 bis 8 bzw. 10 bis 12.

Die Harnblasenprothese für die subkutane Implantation umfasst einen flexiblen, im Wesentlichen schlauchförmigen Flüssigkeitsbehälter, dessen Wand mindestens zwei miteinander verbundene Materialschichten enthält, der eine vordere und eine hintere Seite besitzt, von denen mindestens die vordere Seite stabilisierende Elemente aufweist, der an den seitlichen Enden zwei Öffnungen für den Zulauf von Flüssigkeit aufweist und der mindestens eine Öffnung zum Abfluss von Flüssigkeit aufweist. Dabei sind die Öffnungen stabilisiert oder starr dergestalt ausgebildet, dass sie zur Befestigung von Ventil- oder Schlauchelementen geeignet sind. An den Öffnungen an den seitlichen Enden des Flüssigkeitsbehälters ist jeweils ein Rückschlagventil angeordnet oder es ist an einer der Öffnungen ein Rückschlagventil angeordnet und die andere Öffnung ist mit einem Verschlussstopfen verschlossen. Die äußere Schicht der Flüssigkeitsbehälterwand besteht aus bioinertem Material, vorzugsweise einem organischen Polymer, insbesondere Polyester, vorzugsweise Polyethylenterephthalat (PET), bevorzugt Dacron^{®}. Die innere Schicht der Flüssigkeitsbehälterwand ist glatt und besteht aus einem chemisch inerten Material, vorzugsweise aus einem organischen Polymer, insbesondere Silikon. Erfindungsgemäß sind die Materialschichten der Wand des Flüssigkeitsbehälters miteinander stoffschlüssig verbunden. Zur kontinenten Entleerung der Harnblasenprothese ist an mindestens einer der Öffnungen zum Abfluss von Flüssigkeit ein manuell betätigbares Ventilelement oder ein Schlauchelement, welches ein manuell betätigbares Ventil enthält, angeordnet. Durch manuelle Betätigung des Ventils wird ein kontinentes Ableiten von Flüssigkeit aus dem Flüssigkeitsbehälter gewährleistet. Die mechanische Stabilisierung der Behälterwand und das manuell zu öffnende Ventil verhindern eine unkontrollierte Entleerung der Harnblasenprothese bei versehentlichem Druck auf den Flüssigkeitsbehälter. Da die Harnblasenprothese für die subkutane Implantation ausgestaltet ist, ist eine Bedienung über manuell betätigbare Ventile sichergestellt und ein einfacher operativer Austausch von Bestandteilen der Harnblasenprothese minimalinvasiv möglich.

Der Flüssigkeitsbehälter der erfindungsgemäßen Harnblasenprothese besitzt eine vordere und eine hintere Seite, von denen mindestens die vordere Seite stabilisierende Elemente aufweist. Weiter weist der Flüssigkeitsbehälter an den seitlichen Enden eine oder zwei, vorzugsweise zwei, Öffnungen für den Zulauf von Flüssigkeit und mindestens eine, vorzugsweise eine oder zwei, Öffnungen zum Abfluss von Flüssigkeit auf.

Die Öffnungen sind stabilisiert oder starr dergestalt ausgebildet, dass sie zur Befestigung von Ventil- oder Schlauchelementen geeignet sind.

Um eine subkutane Verträglichkeit der erfindungsgemäßen Harnblasenprothese sicherzustellen, ist der Flüssigkeitsbehälter flexibel gestaltet, damit die subkutane Verbringung nach instrumenteller Expandierung und Etablierung des notwendigen vorzugsweise suprasymphysären Hohlraumes einfach möglich ist. Unter "flexibel" im erfindungsgemäßen Sinn ist zu verstehen, dass der Flüssigkeitsbehälter elastisch und biegsam ist und nach Wegfall einer Krafteinwirkung in die Ursprungsform zurückkehren kann. Bevorzugt ist der Flüssigkeitsbehälter durch Guss- oder Sprayverfahren hergestellt.

Der Flüssigkeitsbehälter eignet sich für die subkutane Implantation. Er besitzt längsseitig zwei Seiten, von denen eine nach der Implantation zur Körperaußenseite zeigt (hierin auch als vordere Seite bezeichnet) und die andere zu den inneren Organen gerichtet ist (hierin auch als hintere Seite bezeichnet). Um die entsprechenden mechanischen Anforderungen nach der Implantation zu erfüllen, enthält mindestens eine der Seiten (die nach der Implantation vordere Seite) stabilisierende Elemente, die in die Wand des Flüssigkeitsbehälters eingearbeitet sind. Ggf. kann die gesamte Wand des Flüssigkeitsbehälters (vordere und hintere Seite) stabilisierende Elemente aufweisen.

Stabilisierende Elemente bewirken, dass das Material oder Werkstoff in ausreichendem Maße biegsam ist, um für die subkutane Implantation geeignet zu sein, gleichwohl gegen äußere Krafteinwirkung in erforderlichem Maße druckstabil ist, so dass keine Zerstörung oder plastische Verformung durch üblicherweise auftretende Krafteinflüsse (z. B. Niesen, Husten, Schlag auf den Bauch) möglich ist. Vorzugsweise werden mechanische Stabilisierungen eingesetzt, um die Druckstabilität zu gewährleisten.

Dadurch ist eine bestimmte Unempfindlichkeit der Harnblasenprothese gegen mechanische Einflüsse von außen gewährleistet. Andererseits wird ein problemloses Entleeren des Flüssigkeitsbehälters der erfindungsgemäßen Harnblasenprothese ermöglicht.

Mechanische Stabilisierungen werden vorzugsweise durch Gerüststrukturen (auch als Stützstrukturen bezeichnet) gewährleistet. Die Gerüststrukturen sind bevorzugt als Spirale, Feder, Rippen oder zylinder- oder quaderförmige Verbindungsstücke (z. B. Stäbe) ausgestaltet. Sie können sowohl aus massivem Material als auch aus Gitter- oder Netzstrukturen bestehen. Vorzugsweise sind die Gerüststrukturen so ausgestaltet, dass diese gleichmäßig verteilt oder durchgehend über die Länge der Harnblasenprothese angeordnet sind. Bevorzugte Materialien für Gerüststrukturen sind Metalle und/oder Polymere. Bevorzugte Stützstrukturen sind Federmetallskelette. Alternativ oder ergänzend zu Gerüststrukturen werden Stabilisierungen durch stabilisierende Elemente vorzugsweise durch Erhöhung der Materialdicke (Dicke der Flüssigkeitsbehälterwand) oder als Rippenskelett aus dem Grundmaterial gewährleistet und/oder indem die Behälterwand mindestens eine weitere Materialschicht als Zwischenschicht zwischen der äußeren und inneren Materialschicht als stabilisierendes Element enthält.

Das Material des Flüssigkeitsbehälters besitzt Memory-Eigenschaften, d. h. dass der Flüssigkeitsbehälter bei nachlassender Krafteinwirkung seine ursprüngliche Gestalt wieder annimmt. Dies ist für die Entleerung der erfindungsgemäßen Harnblasenprothese wichtig, da bei der Entleerung keine Luft in die Harnblasenprothese einströmen soll. Die vordere und hintere Wand des Flüssigkeitsbehälters nähern sich beim Entleeren der Harnblasenprothese an, beim Befüllen durch Zufluss von Harn entfaltet sich der Flüssigkeitsbehälter aufgrund der Memory-Eigenschaften des Materials ohne großen Druckaufwand.

Die Wand des Flüssigkeitsbehälters enthält mindestens zwei miteinander innig verbundene Materialschichten (insbesondere stoffschlüssig, vorzugsweise durch Verkleben der Materialien der einzelnen Schichten), von denen die innere Schicht die Innenwand des Flüssigkeitsbehälters und die äußere Schicht die Außenwand des Flüssigkeitsbehälters bildet. Die Innenwand des Flüssigkeitsbehälters und die Außenwand des Flüssigkeitsbehälters bestehen vorzugsweise aus unterschiedlichen Materialien.

Die Innenwand des Flüssigkeitsbehälters (auch als "Behälterinnenwand" bezeichnet) besteht aus chemisch inertem Material. Unter "chemisch inert" in diesem Zusammenhang sind solche Materialien zu verstehen, die nach der Implantation einer erfindungsgemäßen Harnblasenprothese nicht oder nur in verschwindend geringem Maße mit potentiellen Reaktionspartnern reagieren. Dies bedeutet, dass Materialien der Behälterinnenwand nicht oder nur in verschwindend geringem Maße mit Bestandteilen des Harns reagieren. Die Behälterinnenwand ist glatt, d. h. sie weist vorzugsweise keine Löcher, Risse, Erhebungen oder Unebenheiten auf. Dies ist notwendig, um feste Ablagerungen von Harn an prädestinierten Stellen der Innenwand des Flüssigkeitsbehälters zu vermeiden. Vorzugsweise besteht die Behälterinnenwand aus einem flexiblen Polymer, welches bevorzugt synthetisch oder halbsynthetisch ist. Dieses ist vorzugsweise so ausgewählt, dass es eine niedrige Oberflächenspannung besitzt, so dass es nicht an sich selbst haftet. Besonders bevorzugte Materialien für die Innenwand des Flüssigkeitsbehälters (bzw. die innere Schicht der Flüssigkeitsbehälterwand) sind Silikone (Poly(organo)siloxane).

Die Außenwand des Flüssigkeitsbehälters besteht aus einem bioinerten Material. Unter "bioinert" in diesem Zusammenhang sind solche Materialien zu verstehen, die keine schädigende und immunogene Wirkung auf den menschlichen Körper ausüben und nicht mit umgebenden Substanzen reagieren und dadurch beispielsweise korrodieren. Bioinerte Materialien führen nach der Implantation zu praktisch keiner chemischen und/oder biologischen Wechselwirkung zwischen Material und dem umliegenden Gewebe. Es werden praktisch keine toxischen Substanzen freigesetzt. Vorzugsweise besteht die Behälteraußenwand aus mindestens einem (vorzugsweise organischen) Polymer, welches bevorzugt halbsynthetisch oder synthetisch ist. Besonders bevorzugte Materialien für die Außenwand des Flüssigkeitsbehälters (bzw. die äußere Schicht der Flüssigkeitsbehälterwand) sind Polyethylenterephthalate (PET), ganz besonders bevorzugt Dacron^{®} , Diolen^{®}, Tergal^{®}, Terylene^{®} oder Trevira^{®}. Vorzugsweise besitzt die Außenwand eine raue Oberfläche. In einer Ausgestaltung der Erfindung ist die Außenwand des Flüssigkeitsbehälters aus einer durchgehenden Ummantelung aus einem bioinerten Kunststoff, bevorzugt PET, dargestellt, welche als Überzug auf dem innen liegenden Material oder den innen liegenden Materialschichten vorliegt. So sollen Abstoßungs- und Entzündungsreaktionen verhindert werden, die nur aufgrund einer immunologischen Antwort auf das Oberflächenmaterial erfolgen.

Der Flüssigkeitsbehälter weist eine Gestalt auf, die im Wesentlichen schlauchförmig ist und an den seitlichen Enden eine oder zwei, vorzugsweise zwei Öffnungen aufweist. Unter einer im Wesentlichen schlauchförmigen Form ist zu verstehen, dass der Flüssigkeitsbehälter mit einer rohrförmigen Gestalt ausgebildet ist, wobei der Innendurchmesser des Rohrs an der längsseitigen Behältermitte bevorzugt größer ist, als der Innendurchmesser an den seitlichen Enden. Der Flüssigkeitsbehälter weist vorzugsweise eine symmetrische, insbesondere achsensymmetrische Form auf. Alternativ dazu weist der Flüssigkeitsbehälter vorzugsweise die Form eines Kreisringsegments auf.

Die seitlichen Öffnungen des Flüssigkeitsbehälters dienen dem Zulauf von Flüssigkeit. Das Material des Flüssigkeitsbehälters ist an den Öffnungen stabilisiert oder starr. Bevorzugt sind die Öffnungen mit einer konischen Form ausgestaltet.

Weiter enthält der Flüssigkeitsbehälter mindestens eine, vorzugsweise mindestens eine, vorzugsweise eine oder zwei, Öffnungen zum Abfluss von Flüssigkeit (hierin auch als Abflussöffnungen bezeichnet). Diese sind vorzugsweise in der Mitte des Flüssigkeitsbehälters zwischen den endständigen Öffnungen angeordnet. Besonders bevorzugt sind zwei Öffnungen in der Mitte des Flüssigkeitsbehälters angeordnet, wobei diese zueinander in einem Winkel von etwa 90° versetzt sind. Dies hat den Hintergrund, dass bei Implantation je nach Erfordernis des Patienten der Flüssigkeitsbehälter mit einer Öffnung in die benötigte Richtung (z. B. kaudal oder ventral) angebracht wird. Die andere Öffnung wird in diesem Fall vorzugsweise durch einen Blindstopfen verschlossen.

Sowohl die seitlichen Öffnungen als auch die Öffnungen, die für den Ablauf von Flüssigkeit vorgesehen sind, sind so ausgestaltet, dass sie zur Befestigung von Ventil- oder Schlauchelementen geeignet sind. Dies wird durch Stabilisierung der Öffnungen bewerkstelligt, indem vorzugsweise Metallstrukturen (bevorzugt als Ring) oder andere stabilisierte Materialien (bevorzugt stabilisierende Elemente) für die Ausgestaltung der Öffnungen verwendet werden. Bevorzugte Varianten zur Stabilisierung der Öffnungen liegen in der Auswahl stabilisierter Materialien in Kombination mit einer erhöhten Dicke der Wand des Flüssigkeitsbehälters und dem Einbau von Stützstrukturen, vorzugsweise metallischer Stützstrukturen. Bevorzugt werden die Stützstrukturen in Guss- oder Sprayverfahren in den Flüssigkeitsbehälter eingearbeitet.

An den Öffnungen können Ventil- und Schlauchelemente befestigt werden. Ventilelemente werden direkt an einer Öffnung der erfindungsgemäßen Harnblasenprothese vorzugsweise über Schlauchsteckverbindungen befestigt. Unter "Schlauchelementen" ist hierin ein separater Schlauch oder auch ein Schlauch mit einem daran angeordneten Ventil zu verstehen.

An mindestens einer der seitlichen Öffnungen des Flüssigkeitsbehälters der Harnblasenprothese ist ein Rückschlagventil angeordnet. Rückschlagventile dienen zur Verhinderung der Drucktransmission in die Nieren beim Entleeren des Flüssigkeitsbehälters oder bei auf den Flüssigkeitsbehälter ausgeübtem Druck. Die Befestigung der Ventile wird vorzugsweise durch Schlauchsteckverbindungen bewerkstelligt. Rückschlagventile haben den Vorteil, dass der Rücktritt von Harn aus dem Inneren der Harnblasenprothese in die künstlichen Harnleiter verhindert wird. Als Rückschlagventile sind alle Ventile geeignet, die die Strömungsrichtung der Flüssigkeit (des Harns) so bestimmen, dass nur der Fluss von den Nieren zu dem Flüssigkeitsbehälter erfolgt. Der Durchgang des Harns in die entgegengesetzte Richtung (vom Flüssigkeitsbehälter zu den Nieren) wird durch Rückschlagventile selbsttätig gesperrt. Bevorzugt werden Flatterventile als Rückschlagventile eingesetzt. Flatterventile öffnen in Durchlassrichtung und schließen selbsttätig ohne sonstigen äußeren Antrieb, nur aufgrund von Druckunterschieden auf den beiden Seiten des Ventils. Flatterventile bestehen vorzugsweise aus elastischem Material, dessen Eigenschaften gleichzeitig das Schließen des Ventils sicherstellt und dennoch so steif ist, dass die Ventilfunktion sichergestellt wird. Die Rückschlagventile bestehen bevorzugt aus inertem Kunststoff, insbesondere Silikon.

Erfindungsgemäß ist an mindestens einer Abflussöffnung ein manuell betätigbares Ventilelement oder ein Schlauchelement, welches ein manuell betätigbares Ventil enthält, angeordnet. Für den Fall, dass der Flüssigkeitsbehälter zwei oder mehr Abflussöffnungen aufweist, sind die Öffnungen ohne Ventil- oder Schlauchelement vorzugsweise mit einem Blindstopfen verschlossen. Unter einem "Blindstopfen" ist im Sinne der Erfindung ein Verschlussstopfen zu verstehen, der vorzugsweise aus dem gleichen Material, wie der Flüssigkeitsbehälter besteht. Zumindest sind die Materialien der Innen- und Außenseite des Blindstopfens die gleichen, wie die Innen- und Außenseite des Flüssigkeitsbehälters. Der Blindstopfen ist bevorzugt so gestaltet, dass bei dem Verschluss der Öffnung keine Unebenheiten oder Lücken an Behälterinnenwand und Behälteraußenwand auftreten. Blindstopfen werden ggf. auch an einer der seitlichen Öffnungen des Flüssigkeitsbehälters angeordnet.

Für den Fall, dass ein Ventil über ein Schlauchelement mit der Abflussöffnung des Flüssigkeitsbehälters verbunden ist, ist dieses Ventil vorzugsweise endständig am Schlauchelement angeordnet.

Die an den Abflussöffnungen befestigbaren Ventilkonstruktionen können verschiedenartig gestaltet sein. Sie sind vorzugsweise so ausgestaltet, dass eine manuelle Bedienung des Ventils durch den Patienten möglich ist. Dies geschieht bevorzugt durch ein durch manuellen Druck (auf das Ventil) zu öffnendes Ventil (hierin "Drucköffnungsventil") oder ein katheterisierbares Ventil. Das Ventil ist über die Abflussöffnung direkt oder über einen Schlauch, vorzugsweise über eine aus dem Stand der Technik bekannte künstliche Harnröhre, mit der erfindungsgemäßen Harnblasenprothese verbunden.

Um zu verhindern, dass die Harnblasenprothese bei Überfüllung durch den auftretenden Systemdruck in der Harnblasenprothese (analog zum Harnröhrenverschlussdruck im originären System) beschädigt und damit undicht wird oder, ist das Ventil vorzugsweise so ausgestaltet, dass es bei einem herrschenden Systemdruck, der den maximal zulässigen Systemdruck, für den die Harnblasenprothese ausgelegt ist, übersteigt, automatisch öffnet und den Ablauf von Flüssigkeit ermöglicht. Dadurch wird ein unkontrollierter Austritt von Harn innerhalb des Körpers verhindert.

In einer Ausgestaltung der Erfindung ist direkt an einer Abflussöffnung ein katheterisierbares Ventil angebracht. Die ggf. vorhandene zweite Abflussöffnung ist vorzugsweise mit einem Blindstopfen verschlossen.

Bevorzugt sind die manuell zu betätigenden Ventile selbstverschließend, insbesondere selbstverschließende katheterisierbare Ventile oder selbstverschließende Drucköffnungsventile. Selbstverschließende Ventile sind vorzugsweise aus elastischen Materialien hergestellt.

Das selbstverschließende Drucköffnungsventil ist bevorzugt als Längsschlitz- oder Kreuzschlitzventil ausgestaltet und wird durch Druck von außen auf das Ventil geöffnet. Ein automatisches Schließen des Ventils erfolgt, wenn kein radialer Druck auf das Ventil ausgeübt wird. Kreuzschlitzventile haben zusätzlich den Vorteil, dass eine Öffnung in nahezu jeder Position erfolgen kann, ohne dass auf die Lage des Ventils geachtet werden muss. Bevorzugt ist das Längsschlitz- oder Kreuzschlitzventil als kolbenförmiges elastisches Segment ausgestaltet, welches mit Verschlusskissen oder Dichtungslippen ausgestattet ist und im Inneren den Längsschlitz oder Kreuzschlitz aufweist. Im nichtmanipulierten Zustand schließen die Verschlusskissen oder Dichtungslippen dicht ab und ein Ablauf von Flüssigkeit ist nicht möglich. Nach Zusammendrücken des kolbenförmigen Segments wird der Schlitz geöffnet und ein Ablauf von Flüssigkeit wird ermöglicht. Bei nachlassendem Druck verschließen sich die in ihre elastische Grundform zurückkehrenden Verschlusskissen oder Dichtungslippen selbsttätig.

Ein selbstverschließendes Drucköffnungsventil wird insbesondere in Verbindung mit einem Schlauchelement eingesetzt, welches mit dem Flüssigkeitsbehälter der Harnblasenprothese verbunden ist. Diese Ausgestaltung der Erfindung ist für die Anwendung bei männlichen Patienten besonders geeignet, wobei die Implantation des Schlauchelements in die originäre Harnröhre erfolgt und das Ventil am distalen Ende des Schlauchelements im Penisschaft platziert wird. Durch Zusammendrücken des Penisschaftes erfolgt eine Öffnung des Ventils und die Flüssigkeit wird aus der Harnblasenprothese abgeleitet. Bei nachlassendem Druck verschließt das Ventil selbsttätig.

Das manuell betätigbare Ventil ist bevorzugt aus einem chemisch inerten Material, insbesondere Silikon hergestellt. Bei der Anwendung von Ventilen in Kombination mit Schlauchelementen sind Ventil mit Schlauchelement besonders bevorzugt einstückig ausgebildet. Auf diese Weise entfällt eine nachträgliche Verbindung der beiden Teile, die zu Unebenheiten und Ablagerungen von Bestandteilen des Harns führen kann.

In einer Ausgestaltung der erfindungsgemäßen Harnblasenprothese ist an einer Abflussöffnung ein Schlauchelement angebracht. Die ggf. vorhandene zweite Abflussöffnung ist vorzugsweise mit einem Blindstopfen verschlossen. Das Schlauchelement umfasst eine künstliche Harnröhre (künstliche Urethra) in Kombination mit einem Ventil, vorzugsweise einem Drucköffnungsventil, insbesondere ein selbstverschließendes Längsschlitz- oder Kreuzschlitzventil, welches endständig an der künstlichen Urethra angebracht ist. Künstliche Harnröhren sind aus dem Stand der Technik bekannt. Die künstliche Harnröhre ist mit einem Ende an der Abflussöffnung der Harnblasenprothese verbunden. Am anderen Ende der künstlichen Harnröhre befindet sich ein Ventil.

Alternativ zu bekannten künstlichen Harnröhren können beliebige andere Schlauchelemente an den Abflussöffnungen befestigt werden, die flexibel sind und deren Außenwand aus bioinertem Material besteht.

Das maximale Fassungsvermögen des Flüssigkeitsbehälters einer erfindungsgemäßen Harnblasenprothese wird vorzugsweise nach dem individuellen Bedarf eines Patienten ausgewählt. Es beträgt vorzugsweise maximal 500 ml, besonders bevorzugt 400 bis 500 ml.

Von der Erfindung umfasst ist auch ein Bausatz zur Herstellung einer erfindungsgemäßen Harnblasenprothese, welche für die subkutane Implantation geeignet ist. Der Bausatz umfasst die folgenden Bestandteile:
a) Ein Flüssigkeitsbehälter, wie oben beschrieben. Der Flüssigkeitsbehälter ist flexibel und im Wesentlichen schlauchförmig. Seine Wand besteht aus mindestens zwei miteinander stoffschlüssig verbundenen Materialschichten. Der Flüssigkeitsbehälter besitzt eine vordere und eine hintere Seite, von denen mindestens die vordere Seite stabilisierende Elemente aufweist. Weiter weist der der Flüssigkeitsbehälter an den seitlichen Enden eine oder zwei, vorzugsweise zwei Öffnungen für den Zulauf von Flüssigkeit und mindestens eine Öffnung zum Abfluss von Flüssigkeit auf. Die Öffnungen sind stabilisiert oder starr dergestalt ausgebildet sind, dass sie zur Befestigung von Ventil- oder Schlauchelementen geeignet sind. Die äußere Schicht des Flüssigkeitsbehälters besteht aus bioinertem Material, vorzugsweise einem organischen Polymer, insbesondere Polyester, bevorzugt Polyethylenterephthalat (PET), besonders bevorzugt Dacron^{®}. Die innere Schicht des Flüssigkeitsbehälters ist glatt und besteht aus einem chemisch inerten Material, vorzugsweise aus einem Polymer, insbesondere aus Silikon.
b) Ein oder zwei Rückschlagventile. Diese werden bei der Herstellung der erfindungsgemäßen Harnblasenprothese an den seitlichen Öffnungen des Flüssigkeitsbehälters angeordnet.
c) Ggf. ein Schlauchelement, vorzugsweise eine künstliche Harnröhre. Diese wird bei der Herstellung der erfindungsgemäßen Harnblasenprothese an der Abflussöffnung angeordnet.
d) Ein manuell betätigbares Ventil.
e) Ggf. Blindstopfen. Blindstopfen dienen bei der Herstellung einer erfindungsgemäßen Harnblasenprothese zum Verschluss der Öffnungen, die nicht für Zu- bzw. Ablauf benötigt werden.
f) Ggf. ein Bougierexpander.

Mit einem erfindungsgemäßen Bausatz kann vorteilhaft eine auf den Bedarf des Patienten angepasste Harnblasenprothese direkt vor Implantation zusammengesetzt werden. Die Einzelteile des Bausatzes liegen dabei einzeln oder als Set in steril verpackter Form vor. In bevorzugten Bausätzen ist ein Flüssigkeitsbehälter analog zu a), ein oder zwei Rückschlagventile nach b), ein katheterisierbares Ventil analog zu c) und ein oder zwei Blindstopfen analog zu e) enthalten. In weiteren bevorzugten Bausätzen ist ein Flüssigkeitsbehälter analog zu a), ein oder zwei Rückschlagventile nach b), ein Schlauchelement nach c), ein Drucköffnungsventil nach d) und ein oder zwei Blindstopfen nach e) enthalten. In weiter bevorzugten Bausätzen ist ein Flüssigkeitsbehälter analog zu a) und ein oder zwei Rückschlagventile nach b) und ein katheterisierbares Ventil analog zu c) enthalten.

Das manuell betätigbare Ventil ist wie oben beschrieben ausgestaltet. Bevorzugt ist ein selbstverschließendes Ventil, insbesondere ein katheterisierbares Ventil oder Drucköffnungsventil, im Bausatz enthalten. Ist ein Schlauchelement im erfindungsgemäßen Bausatz enthalten, so ist das Schlauchelement mit Ventil ganz besonders bevorzugt einstückig ausgestaltet.

Erfindungsgemäße Harnblasenprothesen werden wie folgt angewendet: Eine erfindungsgemäße Harnblasenprothese wird einem Patienten subkutan implantiert. Die Öffnungen an den seitlichen Enden des Flüssigkeitsbehälters sind über Rückschlagventile mit künstlichen Harnleitern verbunden, die ebenfalls subkutan implantiert werden. Derartige künstliche Harnleiter sind aus dem Stand der Technik bekannt. Durch die Verwendung derartiger Ventile wird ein Rückfluss von Harn aus der Harnblasenprothese zu den Nieren vermieden.

Für den Fall, dass nur noch eine Niere funktional ist, und daher nur ein künstlicher Harnleiter mit der erfindungsgemäßen Harnblasenprothese verbunden werden kann, wird die zweite seitliche Öffnung des Flüssigkeitsbehälters vorzugsweise mit einem Blindstopfen verschlossen.

Die erfindungsgemäße Harnblasenprothese wird so implantiert, dass die Seite des Flüssigkeitsbehälters, die stabilisierende Elemente aufweist, zur äußeren Körperseite, welche vom Bauchraum weg gerichtet ist, angeordnet ist. Die ggf. ohne zusätzliche Stabilisierung ausgestaltete Seite der Harnblasenprothese befindet sich nach der Implantation auf der inneren, dem Bauchraum und damit den inneren Organen zugewandten Seite.

An einer Abflussöffnung wird ein manuell zu betätigendes Ventilelement oder ein Schlauchelement angebracht. Die ggf. vorhandene andere Abflussöffnung wird vorzugsweise verschlossen, bevorzugt durch einen Blindstopfen. Die Entleerung des erfindungsgemäßen Harnblasenimplantats erfolgt durch Öffnen des Ventils. Für den Fall, dass direkt oder über ein Schlauchelement an die Abflussöffnung ein katheterisierbares Ventil angeordnet wird, erfolgt das Entleeren der Harnblasenprothese durch Katheterisierung. Dabei wird manuell ein Katheter (beispielsweise durch den Bauchnabel) eingeführt, das Ventil dadurch geöffnet und die Harnblasenprothese entleert. Bei Entfernen des Katheters verschließt das Ventil vorzugsweise von selbst. Vorzugsweise wird das katheterisierbare Ventil bei der Implantation im Patienten suprasymphysär-kutan angebracht.

Für den Fall, dass eine künstliche Harnröhre mit einem manuell zu betätigenden Ventil angebracht ist, wird die künstliche Harnröhre vorzugsweise ins Innere der originären Harnröhre so implantiert, dass sie vor der inneren Mündungsöffnung der Harnröhre endet. Die Harnblasenprothese befindet sich nach der Implantation somit vollständig innerhalb des Körpers und hat keinen Kontakt nach außen. Das vorzugsweise am Ende der künstlichen Harnröhre angebrachte Ventil kann vorzugsweise durch Druck von außen auf das Ventil oder durch Katheterisierung geöffnet werden und die Harnblasenprothese dadurch entleert werden. Vorzugsweise sind die eingesetzten Ventile selbstverschließend.

Die Füllung der Harnblasenprothese erfolgt über die seitlichen Öffnungen des Flüssigkeitsbehälters. Dabei wird Harn von den Nieren in den Flüssigkeitsbehälter transportiert. Der Abfluss erfolgt durch manuelle Betätigung eines direkt an der Abflussöffnung angebrachten Ventils oder eines Ventils, welches über ein Schlauchelement mit der Abflussöffnung verbunden ist.

Im ungefüllten Zustand (weder Gas noch Flüssigkeit befinden sich im Inneren) liegen die Behälterinnenwände der vorderen und hinteren Seite des Flüssigkeitsbehälters aufeinander auf. Durch das Einleiten von Harn in die Harnblasenprothese über die Öffnungen für den Zulauf wird die Behälterwand von der einfließenden Flüssigkeit verdrängt und gespannt. Dadurch liegt die Harnblasenprothese im mit Flüssigkeit gefüllten Zustand vorzugsweise rohrförmig vor. Beim Entleeren der Harnblasenprothese über den Abfluss lässt der Innendruck nach, wodurch der Flüssigkeitsbehälter wieder in den anfänglichen Zustand zurückkehrt. Dieser Effekt kann vorteilhaft durch den Druck, welcher durch die umliegenden Organe und Gewebe ausgeübt wird, oder durch manuellen Druck (z. B. Druck mit der Hand auf den Bauch) verstärkt werden.

Die erfindungsgemäße Harnblasenprothese ist so ausgestaltet, dass ein kontinentes Abfließen des Harns aus dem Inneren der Harnblasenprothese möglich ist. Dies wird durch ein Ventil sichergestellt, welches ein manuelles Öffnen und Schließen ermöglicht. Ein Ablassen des Harns ist mit der erfindungsgemäßen Harnblasenprothese daher manuell, vorzugsweise durch den Patienten selbst, durchführbar. Dies erfolgt nicht durch Ausüben von Druck auf den Flüssigkeitsbehälter, wie bei bisher vorgeschlagenen Lösungen, sondern durch manuelle Betätigung eines Ventils, beispielsweise eines katheterisierbaren Ventils oder Drucköffnungsventils. Nach manueller Öffnung des Ventils kann auf den Flüssigkeitsbehälter ausgeübter Druck die Entleerung der implantierten erfindungsgemäßen Harnblasenprothese erleichtern bzw. beschleunigen. Die Öffnung des Ventils erfolgt jedoch nicht durch den auf den Flüssigkeitsbehälter ausgeübten Druck.

Bei der erfindungsgemäßen Lösung wird vermieden, dass einfacher Druck, welcher auf den Flüssigkeitsbehälter ausgeübt wird, eine Entleerung der Harnblasenprothese zur Folge hat. Dadurch wird verhindert, dass die implantierte erfindungsgemäße Harnblasenprothese ungewollt entleert wird (z. B. beim Husten oder Niesen des Patienten). Zur Entleerung muss zunächst manuell das über die zentrale Öffnung des Flüssigkeitsbehälters angebrachte Ventil geöffnet werden. Weiter wird durch die Rückschlagventile verhindert, dass auf den Flüssigkeitsbehälter ausgeübter Druck zu den druckempfindlichen Nieren übertragen wird. Ein Rückfluss von Harn zu den Nieren kann durch die erfindungsgemäße Harnblasenprothese ebenfalls nicht erfolgen.

Durch den stoffschlüssigen Verbund der (mindestens zwei) Materialien, die die Innen- und Außenwand des Flüssigkeitsbehälters beinhalten, wird sichergestellt, dass ein Zusammenziehen des Behälters bei Entleerung erfolgt. Durch die flexiblen und elastischen Eigenschaften des Materials nimmt die Harnblasenprothese bei Befüllung mit Harn ihre im Wesentlichen schlauchförmige Gestalt wieder an. Durch die Verwendung eines chemisch inerten Materials für die Behälterinnenwand wird sichergestellt, dass Ablagerungen von Inhaltsstoffen des Harns im Behälter minimiert werden, welche die Zu- bzw. Abflussöffnungen verengen könnten.

Durch eine erfindungsgemäße Harnblasenprothese wird vermieden, dass vorgenannte Nachteile einer inkontinenten Harnableitung auftreten. Ein entscheidender Vorteil für den Patienten ist, dass bei erfindungsgemäßen Harnblasenprothesen keine extrakorporalen Beutelsysteme notwendig sind, die ständig getragen und entleert werden müssen und eine erhebliche Einschränkung der Lebensqualität bedeuten. Die Bedienung einer erfindungsgemäßen Harnblasenprothese erfordert kein hohes technisches Verständnis, die Entleerung erfolgt durch einfache Ventile manuell vorzugsweise durch den Patienten selbst oder durch Kathetherisierung.

Bei einer erfindungsgemäßen Harnblasenprothese ist vorteilhaft ein einfacher operativer Austausch einzelner Komponenten im Störungsfalle oder bei Komplikationen möglich. Die einzelnen Teile können separat nach Bedarf eingefügt und ggf. im Nutzungsverslauf (beispielsweise bei Defekt durch Verkalkung) ausgetauscht werden. Dies kann im Rahmen einer minimalinvasiven Operation geschehen.

Vorzugsweise wird eine erfindungsgemäße Harnblasenprothese implantiert. Das Implantationsverfahren ist nicht Teil der Erfindung. Ein Verfahren zur subkutanen Implantation einer Harnblasenprothese in einen Patienten umfasst die folgenden Schritte:
- Vornahme mindestens eines seitlichen Schnitts in die Bauchdecke des Patienten,
- Einführen eines Bougierexpanders oder eines vergleichbaren Geräts zur Ausdehnung des erforderlichen, zur Implantation notwendigen subkutanen Raums,
- Einführen einer subkutan implantierbaren Harnblasenprothese in den subkutanen Raum,
- Konnektion subkutan implantierter künstlicher Harnleiter über Rückschlagventile an mindestens einer der Öffnungen des Flüssigkeitsbehälters,
- Konnektion von Schlauchelementen oder Ventilelementen über eine der Öffnungen,
- Vernähen des Materials der Außenwand des Flüssigkeitsbehälters mit den angebrachten Ventil- und/oder Schlauchelementen mit nicht-resorbierbarem Nahtmaterial.

Im Verfahren wird bei dem Patienten mindestens ein seitlicher Schnitt in die Bauchdecke vorgenommen. Dadurch wird ein Bougierexpander oder ein vergleichbares Gerät zur Ausdehnung des erforderlichen, zur Implantation notwendigen subkutanen Raums eingeführt. Nach ausreichender Ausdehnung wird eine erfindungsgemässe Harnblasenprothese subkutan eingeführt.

Bei der Implantation einer erfindungsgemäßen Harnblasenprothese werden weiter die folgenden Schritte vorgenommen: Für den Fall, dass nur eine Seite des Flüssigkeitsbehälters stabilisierende Elemente enthält, geschieht die Implantation so, dass die stabilisierte Seite des Flüssigkeitsbehälters aus der Sicht des Patienten nach außen zeigt. Die Harnblasenprothese wird in der Mitte des Körpers platziert.

Die seitlichen Öffnungen des Flüssigkeitsbehälters werden mit den Enden subkutan implantierter künstlicher Harnleiter verbunden. Zuvor werden Rückschlagventile an den Öffnungen konnektiert (vorzugsweise durch Steckverbindungen). Die Fixation erfolgt durch Vernähen der Ummantelungen von künstlichen Harnleitern und der Oberfläche des Flüssigkeitsbehälters (Außenwand) mit nicht-resorbierbarem Nahtmaterial. Für den Fall, dass nur ein künstlicher Harnleiter angeschlossen wird, wird die nicht genutzte seitliche Öffnung des Flüssigkeitsbehälters mit einem Blindstopfen verschlossen. Die Außenwand des Flüssigkeitsbehälters wird mit dem Außenmaterial des Blindstopfens vernäht.

Die Abflussöffnung in der Mitte des Flüssigkeitsbehälters wird mit einem Ventil- oder Schlauchelement versehen. Die Außenwand des Flüssigkeitsbehälters wird ggf. mit den Außenmaterialien der angeschlossenen Elemente vernäht. Die ggf. vorhandene ungenutzte Abflussöffnung wird mit einem Blindstopfen verschlossen und die äußeren Materialien werden vernäht.

Für den Fall, dass die Harnblasenprothese mit zwei künstlichen Harnleitern verbunden wurde, liegt diese nach der Implantation innerhalb des Körpers U-förmig vor.

Die subkutane Implantation einer Harnblasenprothese bietet die Vorteile, dass eine einfache, minimalinvasive Operation zur Implantation vorgenommen werden kann. Diese sind mit geringeren Risiken für den Patienten verbunden und ermöglichen außerdem eine verkürzte Regenerationszeit nach der Operation. Bei Defekten kann die Harnblasenprothese oder Teile davon einfach operativ ausgetauscht werden.

Ein Verfahren zum Austausch von Teilen einer defekten subkutan implantierten Harnblasenprothese eines Patienten umfasst die folgenden Schritte:
- Vornahme eines mindestens eines Schnitts in die Bauchdecke des Patienten,
- Einführen eines Bougierexpanders oder eines vergleichbaren Geräts zur Ausdehnung des erforderlichen, zur Implantation notwendigen subkutanen Raums,
- Lösen der Nähte des auszutauschenden Teils der subkutan implantierten Harnblasenprothese,
- Austausch des Teils,
- Vernähen der Oberfläche mit nicht-resorbierbarem Nahtmaterial.

Im Verfahren zum Austausch von Teilen einer subkutan implantierten Harnblasenprothese wird eine kleiner, seitlicher, Schnitts durch die Bauchdecke des Patienten vorgenommen. Anschließend werden die Nähte des auszutauschenden Elements der subkutan implantierten Harnblasenprothese gelöst und das gewünschten Element ausgetauscht. Das Vernähen der Oberflächenmaterialien erfolgt mit nicht-resorbierbarem Nahtmaterial.

Anhand folgender Figuren und Ausführungsbeispiele soll die Erfindung näher erläutert werden, ohne die Erfindung auf diese zu beschränken, dabei zeigen:
- Fig. 1.: Erfindungsgemäße Harnblasenprothese mit einem symmetrischen Flüssigkeitsbehälter (1) mit zwei Öffnungen (3) zum Ablauf von Flüssigkeit: a) Vorderansicht, b) seitliche Ansicht, c) Draufsicht.
- Fig. 2: Erfindungsgemäße Harnblasenprothese mit einem symmetrischen Flüssigkeitsbehälter (1) mit einer Öffnung (3) zum Ablauf von Flüssigkeit: a) Vorderansicht, b) seitliche Ansicht, c) Draufsicht.
- Fig. 3: Erfindungsgemäße Harnblasenprothese mit einem Flüssigkeitsbehälter (1) mit zwei Öffnungen (3) zum Ablauf von Flüssigkeit, wobei eine Seite des Flüssigkeitsbehälters stabilisierende Elemente enthält ist: a) Vorderansicht, b) seitliche Ansicht, c) Draufsicht.
- Fig. 4: Erfindungsgemäße Harnblasenprothese mit einem Flüssigkeitsbehälter (1). a) Sicht von vorn und schräg oben, b) seitlicher Schnitt durch die Mitte des Flüssigkeitsbehälters (1).
- Fig. 5: Varianten der Stabilisierung der Öffnungen (2 und/oder 3) durch Kombination unterschiedlicher Materialien.
- Fig. 6: Manuell betätigbares selbstschließendes Drucköffnungsventil mit Längsschlitz.

### Beispiel: Harnblasenprothese für subkutane Implantation

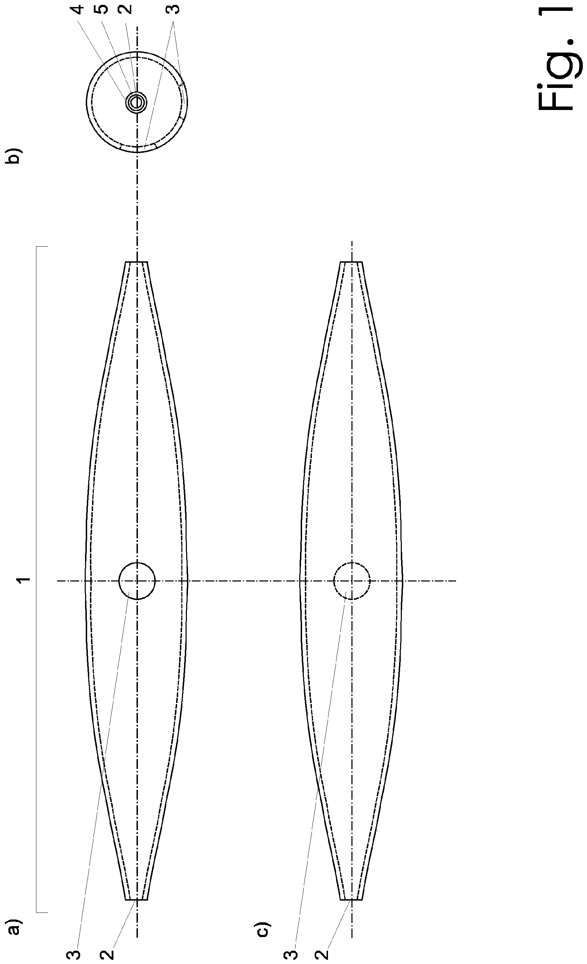

Der Aufbau erfindungsgemäßer Harnblasenprothesen ist in den Figuren 1 bis 4 dargestellt.

**Fig. 1** zeigt eine erfindungsgemäße Harnblasenprothese mit einem symmetrischen Flüssigkeitsbehälter (1) mit zwei Öffnungen (3) zum Ablauf von Flüssigkeit. Der schlauchförmige Flüssigkeitsbehälter (1) besitzt zwei seitliche Öffnungen (2) für den Zulauf von Flüssigkeit und mittig zwei Öffnungen (3) für den Ablauf von Flüssigkeit. Diese Öffnungen (3) sind um etwa 90° zueinander versetzt. Die Wand des Flüssigkeitsbehälters (1) besteht aus zwei Materialschichten, von denen eine die Innenwand (5) und eine die Außenwand (4) bildet. Der Flüssigkeitsbehälter (1) besitzt eine vordere und hindere Seite mit stabilisierenden Elementen.

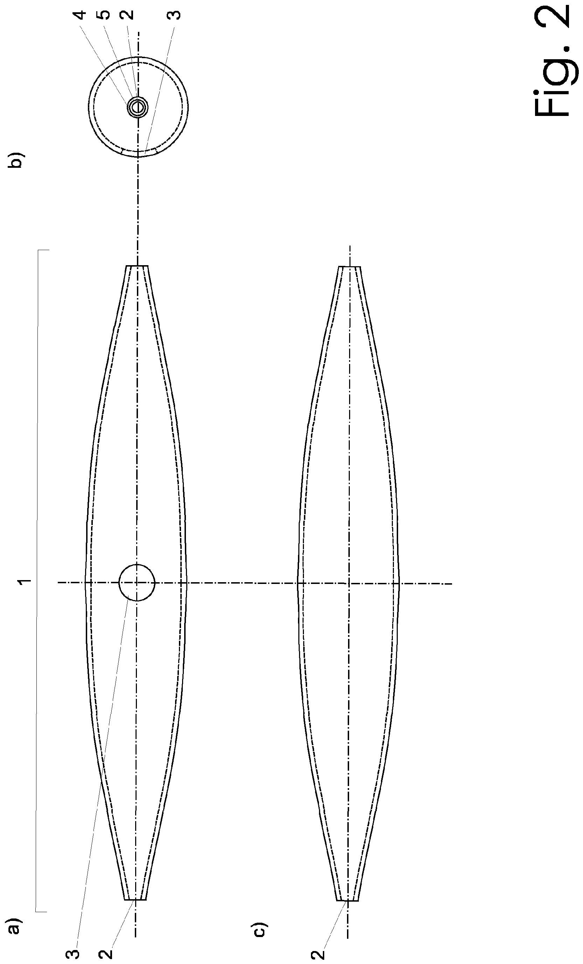

**Fig. 2** zeigt eine erfindungsgemäße Harnblasenprothese mit einem symmetrischen Flüssigkeitsbehälter (1) mit einer Öffnung (3) zum Ablauf von Flüssigkeit. Der schlauchförmige Flüssigkeitsbehälter (1) besitzt zwei seitliche Öffnungen (2) für den Zulauf von Flüssigkeit und mittig eine Öffnung (3) für den Ablauf von Flüssigkeit. Die Wand des Flüssigkeitsbehälters (1) besteht aus zwei Materialschichten, von denen eine die Innenwand (5) und eine die Außenwand (4) bildet. Der Flüssigkeitsbehälter (1) besitzt eine vordere und hindere Seite mit stabilisierenden Elementen.

Zur Stabilisierung sind vorzugsweise Gerüststrukturen in die Wand des Flüssigkeitsbehälters eingearbeitet und/oder die Materialdicke der Behälterwand ist erhöht.

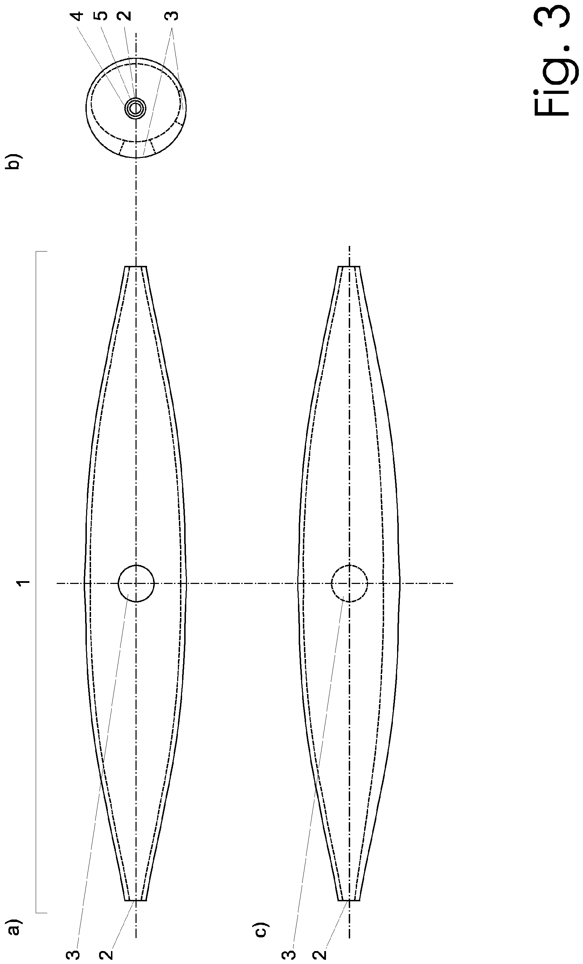

**Fig. 3** zeigt eine erfindungsgemäße Harnblasenprothese mit einem Flüssigkeitsbehälter (1) mit zwei Öffnungen (3) zum Ablauf von Flüssigkeit, wobei eine Seite des Flüssigkeitsbehälters stabilisierende Elemente aufweist. Dies ist durch die höhere Wanddicke an einer Seite des Flüssigkeitsbehälters gekennzeichnet. Der schlauchförmige Flüssigkeitsbehälter (1) besitzt zwei seitliche Öffnungen (2) für den Zulauf von Flüssigkeit und mittig zwei Öffnungen (3) für den Ablauf von Flüssigkeit. Diese sind um etwa 90° zueinander versetzt. Die Wand des Flüssigkeitsbehälters (1) besteht aus zwei Materialschichten, von denen eine die Innenwand (5) und eine die Außenwand (4) bildet. Auch diese Variante ist mit der Ausgestaltung mit einer Öffnung (3) kombinierbar.

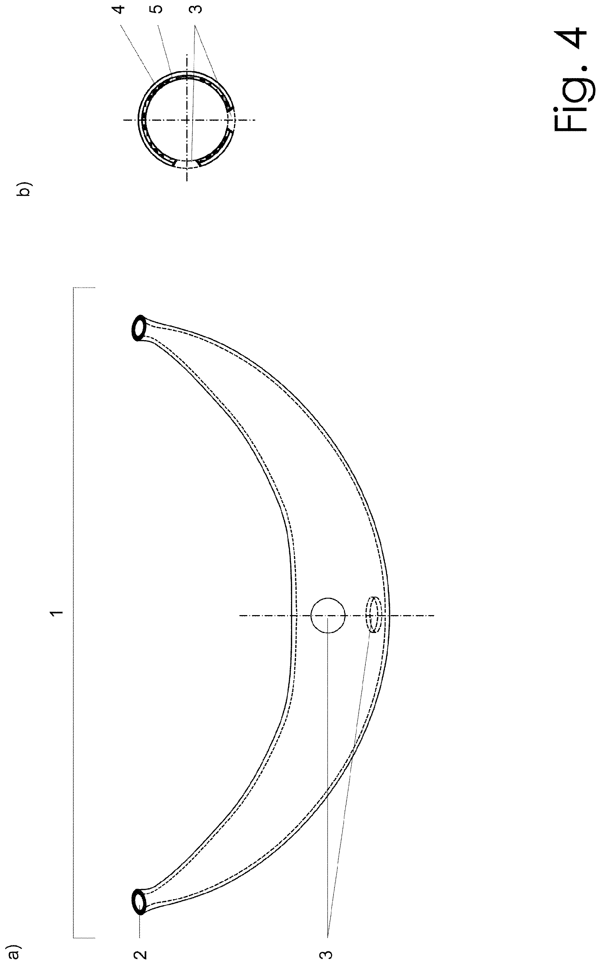

**Fig. 4** zeigt eine Außenansicht einer erfindungsgemäßen Harnblasenprothese (Fig. 4a) sowie einen seitlichen Schnitt durch die Mitte der abgebildeten Harnblasenprothese. Abgebildet ist der schlauchförmige Flüssigkeitsbehälter (1), der zwei seitliche Öffnungen (2) für den Zulauf von Flüssigkeit und mittig zwei Öffnungen (3) für den Ablauf von Flüssigkeit besitzt. Die Öffnungen (3) sind um etwa 90° zueinander versetzt (Fig. 4a). Dies ist ebenfalls im seitlichen Schnitt auf Fig. 4b zu erkennen. Die Wand des Flüssigkeitsbehälters (1) besteht aus zwei Materialschichten, von denen eine die Innenwand (5) und eine die Außenwand (4) bildet (Fig. 4b).

Die Harnblasenprothese ist schlauchförmig mit einem Fassungsvermögen von ca. 500 ml ausgestaltet und besitzt nach der Implantation und Konnektion mit zwei künstlichen Harnleitern eine U-förmige Gestalt (Fig. 4 a).

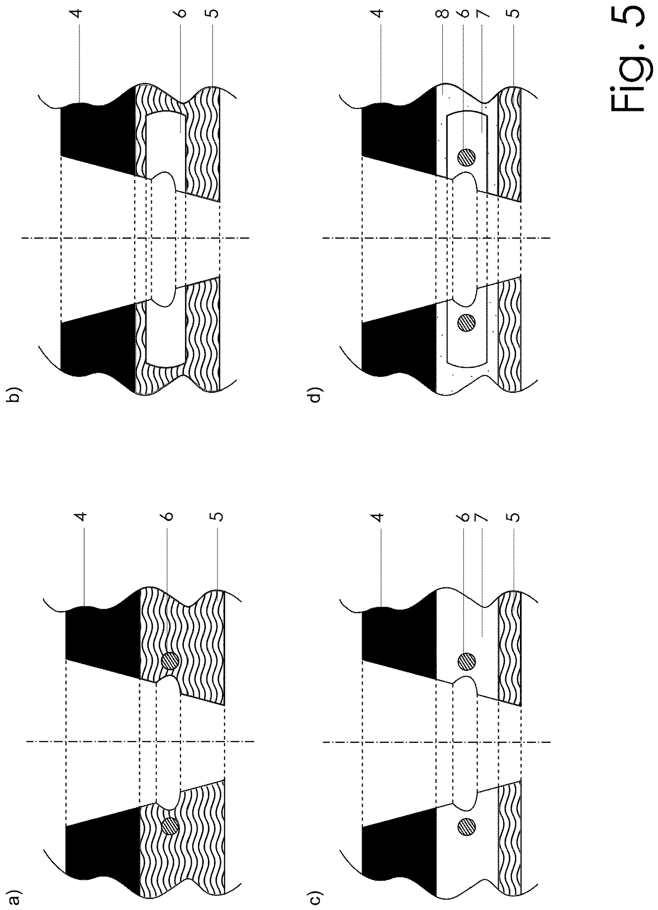

Die Wand des Flüssigkeitsbehälters besteht aus mindestens zwei miteinander innig verbundenen Materialschichten, wobei die innere Schicht die Innenwand (5) und die äußere Schicht, die Außenwand (4) bildet. Ggf. können weitere Materialschichten (8) vorhanden sein (Fig. 5).

Die Innenwand (5) des Flüssigkeitsbehälters (1) und die Außenwand (4) des Flüssigkeitsbehälters (1) bestehen aus unterschiedlichen Materialien. Die Innenwand (5) des Flüssigkeitsbehälters (1) ist chemisch inert, glatt und besteht aus Silikon. Die Außenwand (4) ist bioinert und besteht aus Polyethylenterephthalat (PET), bevorzugt Dacron^{®}.

Die Öffnungen (2, 3) eines Flüssigkeitsbehälters sind stabilisiert. An den Öffnungen (2) ist jeweils ein Rückschlagventil angeordnet (nicht dargestellt).

Ausgestaltungen der Öffnungen (2, 3) erfindungsgemäßer Harnblasenprothesen sind in Fig. 5 abgebildet, ohne die Möglichkeiten auf diese Varianten zu beschränken. Die Öffnungen (2, 3) sind vorzugsweise konisch ausgestaltet. Die Öffnungen (2, 3) sind so gestaltet, dass sie sich zur Befestigung von Ventil- und/oder Schlauchelementen eignen. Vorzugsweise geschieht dies durch mindestens eine Nut in der Öffnung (2, 3).

Fig. 5 a) zeigt eine Stabilisierung durch einen Ring (6), der komplett in die innere Materialschicht (5) eingelassen ist. Fig. 5 b) zeigt eine Stabilisierung durch eine Ringstruktur (6), die in die innere Materialschicht eingelassen ist und in deren Material eine Nut eingelassen ist. Fig. 5 c) zeigt eine Stabilisierung durch einen Ring (6), die in eine stabilisierende Materialschicht (7) eingelassen ist. Fig. 5 d) zeigt eine Stabilisierung durch einen Ring (6), der in eine stabilisierte Ringstruktur (9) eingelassen ist, wobei die Ringstruktur (9) in einer Materialschicht (8) eingelassen ist, die vorzugsweise nicht die Außenwand (4) des Flüssigkeitsbehälters ist.

Die Harnblasenprothese wird einem Patienten subkutan implantiert und dient zum Ersatz der originären Harnblase. Die Öffnungen (2) werden mit zwei künstlichen Harnleitern verbunden, welche ebenfalls subkutan implantiert werden und zur Ableitung des Harns von den Nieren dienen. Der Harn wird von den Nieren über die künstlichen Harnleiter über die Öffnungen (2) in den Flüssigkeitsbehälter (1) eingetragen. An den Öffnungen werden Rückschlagventile montiert, die verhindern, dass Harn aus dem Flüssigkeitsbehälter (1) in die (über die Rückschlagventile mit dem Flüssigkeitsbehälter (1) verbundenen) künstlichen Harnleiter und damit zu den Nieren zurückgeführt wird. Das Eintragen der Flüssigkeit kann dadurch nur in Richtung des Flüssigkeitsbehälters (1) erfolgen. Für den Fall, dass nur ein künstlicher Harnleiter mit dem Flüssigkeitsbehälter (1) konnektiert (verbunden) wird, wird die andere Öffnung (2) mit einem Blindstopfen dicht verschlossen und die Außenwand des Flüssigkeitsbehälters (1) wird mit dem Außenmaterial des Blindstopfens vernäht.

Durch das Eindringen der Harnflüssigkeit in den Flüssigkeitsbehälter entfaltet sich die Wand des Flüssigkeitsbehälters (1), so dass die Harnblasenprothese im gefüllten Zustand rohrförmig vorliegt.

Die Entleerung der Harnblasenprothese erfolgt über eine der Öffnungen (3). An einer der Öffnungen (3) wird dafür ein Ventil- oder Schlauchelement montiert, an der ggf. vorhandenen zweiten Öffnung (3) wird ein Blindstopfen angebracht, der diese Öffnung (3) dicht verschließt. Die Materialien der Außenwand (4) des Flüssigkeitsbehälters (1) werden mit dem Außenmaterial des Blindstopfens vernäht. Die Entleerung des Flüssigkeitsbehälters (1) erfolgt durch vorzugsweise manuelles Öffnen des Ventils an der Öffnung (3). Beispielhafte Ventile sind katheterisierbare Ventile, welche durch Einführen eines Katheters manuell geöffnet werden. Andere geeignete Ventile sind Drucköffnungsventile, welche durch Druck geöffnet werden und bei nachlassendem Druck selbsttätig schließen.

### Beispielhaft seien zwei Implantationsvarianten mit unterschiedlichen Anschlüssen an die Öffnung (3) genannt:

Einem männlichen Patienten mit noch vorhandener originärer Harnröhre wird eine erfindungsgemäße Harnblasenprothese implantiert. Dabei zeigt die Öffnung (3) in kaudale Richtung. Dies ermöglicht das Konnektieren einer künstlichen Harnröhre an die Öffnung (3), welche subkutan und perineal in die originäre Harnröhre implantiert wird. Am anderen Ende der künstlichen Harnröhre ist ein selbstverschließendes Drucköffnungsventil angeordnet, welches innerhalb des Penisschaftes platziert wird. Durch Zusammendrücken des Penisschaftes durch den Patienten erfolgt die Öffnung des Ventils und der Harn wird aus der Harnblasenprothese abgeleitet. Bei nachlassendem Druck verschließt das Ventil selbsttätig.

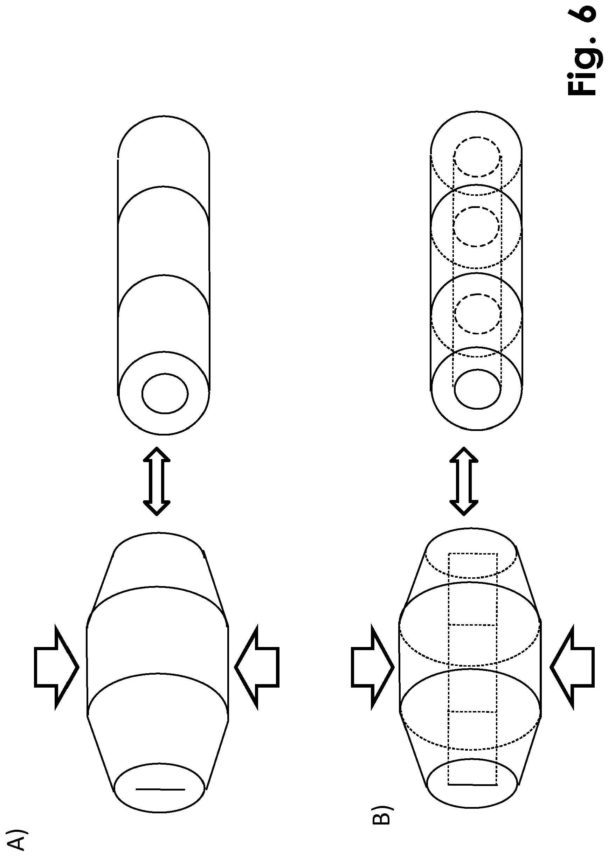

Der Aufbau und Funktionsmechanismus eines manuell betätigbaren Drucköffnungsventils mit Längsschlitz ist in Fig. 6 dargestellt. Das kolbenförmig ausgebildete Ventil umfasst elastische Verschlusskissen aus einem chemisch inerten Kunststoff, insbesondere Silikon, welche im unmanipulierten Zustand dicht abschließen. Durch Ausübung von Druck außen auf das Ventil entlang der Längsrichtung des Schlitzes werden die Verschlusskissen zu einer nahezu kreisrunden Öffnung geöffnet, so dass der Harn abgeleitet werden kann (links in der Abbildung dargestellt). Nach Beendigung des Zusammendrückens verschließen die in ihre elastische Grundform zurückkehrenden Verschlusskissen selbsttätig. Ein ungewünschtes Ableiten des Harns ist in diesem Zustand nicht möglich.

In einer weiteren Variante wird einem weiblichen Patienten mit defekter Harnröhre eine erfindungsgemäße Harnblasenprothese aus implantiert. Dabei zeigt die Öffnung (3) in ventrale Richtung. Dies ermöglicht die Ableitung durch ein an die Öffnung (3) angeschlossenes katheterisierbares Ventil, welches beispielsweise so implantiert wird, dass die Katheterisierung über den Bauchnabel erfolgen kann.

Beim Entleeren der subkutan implantierten erfindungsgemäßen Harnblasenprothese dringt vorzugsweise keine Luft in die Prothese ein, so dass die Behälterinnenwand entspannt ist.

Durch die subkutane Implantation können bei Defekten vorteilhaft sowohl die Harnblasenprothese als auch Einzelteile davon in einer einfachen Operation ausgetauscht werden.

### Bezugsnummern

(1) Flüssigkeitsbehälter
(2) Öffnung (für Zulauf von Flüssigkeit)
(3) Öffnung (für Abfluss von Flüssigkeit)
(4) Außenwand
(5) Innenwand
(6) Gerüststruktur
(7) stabilisierende Schicht
(8) Materialschicht der Wand des Flüssigkeitsbehälters
(9) Gerüststruktur

## Patentansprüche

1. Harnblasenprothese für die subkutane Implantation, umfassend einen flexiblen, im Wesentlichen schlauchförmigen Flüssigkeitsbehälter (1),
- dessen Wand mindestens zwei Materialschichten enthält,
- der eine vordere und eine hintere Seite besitzt,
- der an den seitlichen Enden zwei Öffnungen (2) für den Zulauf von Flüssigkeit aufweist und
- der mindestens eine Öffnung (3) zum Abfluss von Flüssigkeit aufweist, wobei
o die Öffnungen (2 und 3) stabilisiert oder starr dergestalt ausgebildet sind, dass sie zur Befestigung von Ventil- oder Schlauchelementen geeignet sind, und
o an den Öffnungen (2) jeweils ein Rückschlagventil angeordnet ist oder an einer der Öffnungen (2) ein Rückschlagventil angeordnet ist und die andere Öffnung (2) mit einem Verschlussstopfen verschlossen ist,
- wobei die äußere Schicht (4) der Flüssigkeitsbehälterwand aus bioinertem Material, vorzugsweise einem organischen Polymer, insbesondere Polyester, vorzugsweise Polyethylenterephthalat, bevorzugt Dacron^{®} besteht, und
- die innere Schicht (5) der Flüssigkeitsbehälterwand glatt ist und aus einem chemisch inerten Material, vorzugsweise aus einem organischen Polymer, insbesondere Silikon besteht,
**dadurch gekennzeichnet, dass**
- die Materialschichten der Wand des Flüssigkeitsbehälters (1) miteinander stoffschlüssig verbunden sind,
- an mindestens einer der Öffnungen (3) ein manuell betätigbares Ventilelement oder ein Schlauchelement, welches ein manuell betätigbares Ventil enthält, angeordnet ist.

2. Harnblasenprothese nach Anspruch 1, **dadurch gekennzeichnet, dass** das manuell betätigbare Ventil selbstverschließend ist.

3. Harnblasenprothese nach Anspruch 1 oder 2, **dadurch gekennzeichnet, dass** das manuell betätigbare Ventil ein katheterisierbares Ventil ist.

4. Harnblasenprothese nach Anspruch 1 oder 2, **dadurch gekennzeichnet, dass** das Ventilelement oder das Schlauchelement ein von außen durch Druckausübung auf das Ventil zu öffnendes manuell zu betätigendes Ventil umfasst.

5. Harnblasenprothese nach Anspruch 4, **dadurch gekennzeichnet, dass** manuell zu betätigende Ventil als selbstschließendes Kreuzschlitz- oder Längsschlitzventil ausgebildet ist.

6. Harnblasenprothese nach einem der vorhergehenden Ansprüche, **dadurch gekennzeichnet, dass** der Flüssigkeitsbehälter als stabilisierende Elemente eine eingearbeitete Stützstruktur, vorzugsweise eine Metallstruktur, insbesondere ein Federmetallskelett aufweist.

7. Harnblasenprothese nach einem der vorhergehenden Ansprüche, **dadurch gekennzeichnet, dass** der Flüssigkeitsbehälter als stabilisierende Elemente, mindestens eine Materialschicht als Zwischenschicht zwischen der äußeren und inneren Materialschicht aufweist.

8. Harnblasenprothese nach einem der vorhergehenden Ansprüche, **dadurch gekennzeichnet, dass** der Flüssigkeitsbehälter ein Fassungsvermögen von maximal 500 ml, vorzugsweise 400 bis 500 ml besitzt.

9. Bausatz zur Herstellung einer Harnblasenprothese, enthaltend die folgenden Bestandteile:
a) ein flexibler, im Wesentlichen schlauchförmiger Flüssigkeitsbehälter (1), dessen Wand mindestens zwei miteinander stoffschlüssig verbundene Materialschichten enthält, der eine vordere und eine hintere Seite besitzt, von denen mindestens die vordere Seite stabilisierende Elemente aufweist, der an den seitlichen Enden zwei Öffnungen (2) für den Zulauf von Flüssigkeit aufweist und der mindestens eine Öffnung (3) zum Abfluss von Flüssigkeit aufweist, wobei die Öffnungen (2 und 3) stabilisiert oder starr dergestalt ausgebildet sind, dass sie zur Befestigung von Ventil- oder Schlauchelementen geeignet sind, wobei die äußere Schicht (4) der Flüssigkeitsbehälterwand aus bioinertem Material, vorzugsweise einem organischen Polymer, insbesondere Polyester, vorzugsweise Polyethylenterephthalat, bevorzugt Dacron^{®} besteht, und die innere Schicht (5) der Flüssigkeitsbehälterwand glatt ist und aus einem chemisch inerten Material, vorzugsweise aus einem organischen Polymer, insbesondere Silikon besteht,
b) ein oder zwei Rückschlagventile,
c) ggf. ein Schlauchelement, vorzugsweise eine künstliche Harnröhre,
d) ein manuell betätigbares Ventil,
e) ggf. Blindstopfen,
f) ggf. ein Bougierexpander.

10. Bausatz nach Anspruch 9, wobei das manuell betätigbare Ventil selbstverschließend ist.

11. Bausatz nach Anspruch 9 oder 10, wobei das manuell betätigbare Ventil ein katheterisierbares Ventil oder ein von außen durch Druckausübung auf das Ventil zu öffnendes manuell betätigbares Ventil ist.

12. Bausatz nach einen der Ansprüche 9 bis 11, wobei das manuell betätigbare Ventil als selbstschließendes Kreuzschlitz- oder Längsschlitzventil ausgebildet ist, welches durch Druckausübung von außen auf das Ventil zu öffnen ist.

## Claims

1. A urinary bladder prosthesis for subcutaneous implantation, comprising a flexible, essentially tubular liquid container (1),
- the wall of which comprises at least two layers of material,
- having a front side and a rear side,
- having two openings (2) for the inflow of fluid on the lateral ends and
- having at least one opening (3) for the outflow of liquid, wherein
○ the openings (2 and 3) are stabilized or are designed to be rigid, such that they are suitable for fastening valve elements or tubular elements, and
○ a nonreturn valve is disposed at each of the openings (2) or a nonreturn valve is disposed at one of the openings (2) and the other opening (2) is closed with a closing stopper,
- wherein the exterior layer (4) of the liquid container wall is made of a bioinert material, preferably an organic polymer, in particular polyester, preferably polyethylene terephthalate, preferably Dacron®, and
- the interior layer (5) of the liquid container wall is smooth and is made of a chemically inert material, preferably an organic polymer, in particular silicone,
**characterized in that**
- the material layers of the wall of the liquid container (1) are physically bonded to one another,
- a manually operable valve element or a tubular element, which includes a manually operable valve, is disposed on at least one of the openings (3).

2. The urinary bladder prosthesis according to claim 1, **characterized in that** the manually operable valve is self-closing.

3. The urinary bladder prosthesis according to claim 1 or 2, **characterized in that** the manually operable valve is catheterizable valve.

4. The urinary bladder prosthesis according to claim 1 or 2, **characterized in that** the valve element or the tubular element comprises a valve to be operated manually and to be opened by exerting pressure externally on the valve.

5. The urinary bladder prosthesis according to claim 4, **characterized in that** the valve to be operated manually is designed as a self-closing cross-slot valve or longitudinal slot valve.

6. The urinary bladder prosthesis according to any one of the preceding claims, **characterized in that** the liquid container has an incorporated support structure, preferably a metal structure, in particular a spring metal skeleton as stabilizing elements.

7. The urinary bladder prosthesis according to any one of the preceding claims, **characterized in that** the liquid container has at least one layer of material as an intermediate layer between the exterior and interior layers of material as stabilizing elements.

8. The urinary bladder prosthesis according to any one of the preceding claims, **characterized in that** the liquid container has a capacity of max. 500 mL, preferably 400 to 500 mL.

9. A kit for producing a urinary bladder prosthesis, comprising the following components:
a) a flexible, essentially tubular liquid container from (1), the wall of which comprises at least two layers of material physically bonded to one another, having a front side and a rear side, of which at least the front side has stabilizing elements having two openings (2) for the inflow of liquid on the lateral ends and having at least one opening (3) for outflow of liquid, wherein the openings (2 and 3) are stabilized or are designed to be rigid, such that they are suitable for fastening valve elements or tubular elements, wherein the exterior layer (4) of the liquid container wall is made of a bioinert material, preferably an organic polymer, in particular polyester, preferably polyethylene terephthalate, preferably Dacron®, and the interior layer (5) of the liquid container wall is smooth and is made of a chemically inert material, preferably an organic polymer, in particular silicone,
b) one or two nonreturn valves,
c) optionally a tubular element, preferably an artificial urethra,
d) a manually operable valve,
e) optionally blank stoppers,
f) optionally an expander bougie.

10. The kit according to claim 9, wherein the manually operable valve is self-closing.

11. The kit according to claim 9 or 10, wherein the manually operable valve is a catheterizable valve or a manually operable valve to be opened by pressure exerted on the valve from the outside.

12. The kit according to any one of claims 9 to 11, wherein the manually operable valve is designed as a self-closing cross-slot or longitudinal-slot valve, which is to be opened by applying pressure to the valve from the outside.

## Revendications

1. Prothèse de vessie destinée à l'implantation sous-cutanée, comprenant un réservoir à liquide (1) flexible, de forme sensiblement tubulaire,
- dont la paroi contient au moins deux couches de matières,
- qui présente une face avant et une face arrière,
- qui sur les extrémités latérales comporte deux orifices (2) pour l'arrivée de liquide et
- qui comporte au moins un orifice (3) pour l'écoulement de liquide,
○ les orifices (2 et 3) étant stabilisés ou rigides, de sorte à être adaptés pour la fixation d'éléments formant soupapes ou d'éléments tubulaires et
○ sur les orifices (2) étant placé chaque fois un clapet antiretour ou sur l'un des orifices (2) étant placé un clapet antiretour et l'autre orifice (2) étant fermé par un bouchon obturateur,
- la couche extérieure (4) de la paroi du réservoir à liquide étant constituée d'une matière bioinerte, de préférence d'un polymère organique, notamment de polyester, de préférence de téréphtalate de polyéthylène, de préférence de Dacron® et
- la couche intérieure (5) de la paroi du réservoir à liquide étant lisse et étant constituée d'une matière chimiquement inerte, de préférence d'un polymère organique, notamment de silicone,
**caractérisée en ce que**
- les couches de matières de la paroi du réservoir à liquide (1) sont reliées l'une à l'autre par matière,
- sur au moins l'un des orifices (3) est placé un élément formant soupape ou un élément tubulaire, lequel contient une soupape à actionnement manuel.

2. Prothèse de vessie selon la revendication 1, **caractérisée en ce que** la soupape à actionnement manuel est auto-obturatrice.

3. Prothèse de vessie selon la revendication 1 ou 2, **caractérisée en ce que** la soupape à actionnement manuel est une soupape cathérisable.

4. Prothèse de vessie selon la revendication 1 ou 2, **caractérisée en ce que** l'élément formant soupape ou l'élément tubulaire comprend une soupape à actionnement manuel par l'extérieur, qui s'ouvre par l'exercice d'une pression sur la soupape.

5. Prothèse de vessie selon la revendication 4, **caractérisée en ce que** la soupape à actionnement manuel est conçue en tant que soupape auto-obturatrice à empreinte cruciforme ou à fente oblongue.

6. Prothèse de vessie selon l'une quelconque des revendications précédentes, **caractérisée en ce qu'**en tant qu'éléments stabilisateurs, le réservoir à liquide comporte une structure de soutien incorporée, de préférence une structure métallique, notamment un squelette en métal à ressort.

7. Prothèse de vessie selon l'une quelconque des revendications précédentes, **caractérisée en ce qu'**en tant qu'éléments stabilisateurs, le réservoir à liquide comporte une couche de matière en tant que couche intermédiaire entre les couches de matières extérieure et intérieure.

8. Prothèse de vessie selon l'une quelconque des revendications précédentes, **caractérisée en ce que** le réservoir à liquide a une capacité d'un maximum de 500 ml, de préférence de 400 à 500 ml.

9. Kit destiné à fabriquer une prothèse de vessie, contenant les composants suivants :
a) un réservoir à liquide (1) flexible, de forme sensiblement tubulaire dont la paroi contient au moins deux couches de matières reliées l'une à l'autre par matière, qui présente une face avant et une face arrière, dont au moins la face avant comporte des éléments stabilisateurs, qui sur les extrémités latérales comporte deux orifices (2) pour l'arrivée de liquide et qui comporte au moins un orifice (3) pour l'écoulement de liquide, les orifices (2 et 3) étant stabilisés ou rigides, de sorte à être adaptés pour la fixation d'éléments formant soupapes ou d'éléments tubulaires, la couche extérieure (4) de la paroi du réservoir à liquide étant constituée d'une matière bio-inerte, de préférence d'un polymère organique, notamment de polyester, de préférence de téréphtalate de polyéthylène, de préférence de Dacron® et la couche intérieure (5) de la paroi du réservoir à liquide étant lisse et étant constituée d'une matière chimiquement inerte, de préférence d'un polymère organique, notamment de silicone,
b) un ou deux clapets antiretour,
c) le cas échéant, un élément tubulaire, de préférence un urètre artificiel,
d) une soupape à actionnement manuel,
e) le cas échéant, des bouchons obturateurs,
f. le cas échéant, un expandeur de mouvement.

10. Kit selon la revendication 9, la soupape à actionnement manuel étant auto-obturatrice.

11. Kit selon la revendication 9 ou 10, la soupape à actionnement manuel étant une soupape cathérisable ou une soupape à actionnement manuel par l'extérieur, qui s'ouvre par l'exercice d'une pression sur la soupape.

12. Kit selon l'une quelconque des revendications 9 à 11, la soupape à actionnement manuel étant conçue en tant que soupape auto-obturatrice à empreinte cruciforme ou à fente oblongue qui s'ouvre par l'exercice d'une pression par l'extérieur sur la soupape.
